# FASCICULE DE BREVET EUROPEEN

(11) **EP 1 446 373 B1**
(45) Date de publication et mention de la délivrance du brevet: **04.08.2010**
(21) Numéro de dépôt: 02803436.1
(22) Date de dépôt: 18.11.2002
(51) Int. Cl.: C07B 63/04, C07D 493/04, C07D 307/20

(54) **PROCEDE DE PREPARATION D'UNE COMPOSITION CONTENANT AU MOINS UN PRODUIT DE DESHYDRATATION INTERNE D'UN SUCRE HYDROGENE**
VERFAHREN ZUR HERSTELLUNG EINER ZUSAMMENSETZUNG ENTHALTEND MINDENSTENS EIN DEHYDRATISIERUNGSPRODUKT EINES HYDROGENISIERTEN ZUCKERS
METHOD FOR PREPARING A COMPOSITION CONTAINING AT LEAST AN INTERNAL DEHYDRATING PRODUCT OF A HYDROGENATED SUGAR

(30) Priorité: 20.11.2001 FR 0115018
(43) Date de publication de la demande: 18.08.2004
(73) Titulaire: Roquette Frères, 62136 Lestrem (FR)
(72) Inventeur: FUERTES, Patrick, F-59130 Lambersart (FR)
(74) Mandataire: Nargolwalla, Cyra
(86) Numéro de dépôt international: PCT/FR2002/003935
(87) Numéro de publication internationale: WO 2003/043959

(56) Documents cités:
- WO-A-00/14081
- WO-A-00/41985
- US-A- 3 160 641
- PATENT ABSTRACTS OF JAPAN vol. 1998, no. 03, 27 février 1998 (1998-02-27) & JP 09 286787 A (NEW JAPAN CHEM CO LTD), 4 novembre 1997 (1997-11-04)

## Description

La présente invention a pour objet un nouveau procédé de préparation d'une composition contenant au moins un produit de déshydratation interne d'un sucre hydrogéné.

Elle concerne également l'utilisation de la composition ainsi préparée dans la préparation de produits ou mélanges, polymériques ou non, biodégradables ou non, destinés en particulier aux industries chimiques, pharmaceutiques, cosmétologiques ou alimentaires.

Enfin, la présente invention a également pour objet, en tant que produit nouveau susceptible d'être obtenu selon ledit procédé, une composition du genre en question présentant des caractéristiques particulières, notamment en termes de stabilité, de pureté et/ou de teneur en certaines espèces, stabilisantes ou non.

Par « sucre hydrogéné » au sens de la présente invention, on entend en particulier :
- les hexitols tels que, par exemple, le sorbitol, le mannitol, l'iditol et le galactitol,
- les pentitols tels que, par exemple, l'arabitol, le ribitol et le xylitol, et
- les tétritols tels que, par exemple, l'érythritol.

Par « produit de déshydratation interne », on entend tout produit résultant, d'une manière quelconque, en une ou plusieurs étapes, de l'enlèvement d'une ou de plusieurs molécules d'eau au niveau de la structure interne originelle d'un sucre hydrogéné tel que ceux mentionnés ci-avant.

Il peut s'agir avantageusement de produits de déshydratation interne d'hexitols, en particulier de dianhydro hexitols ou « isohexides » comme l'isosorbide (1,4 - 3,6 dianhydro sorbitol), l'isomannide (1,4 - 3,6 dianhydro mannitol) ou l'isoidide (1,4 - 3,6 dianhydro iditol).

Parmi ces sucres hydrogénés doublement déshydratés, l'isosorbide est aujourd'hui celui pour lequel on développe, pour le moins on envisage, le plus d'applications industrielles. Celles-ci concernent notamment :
- la préparation de 2-nitrate, 5-nitrate ou 2,5 dinitrate d'isosorbide, utiles dans le traitement thérapeutique de maladies, notamment cardiaques et/ou vasculaires - comme décrit dans le brevet US 4,371,703,
- la préparation de dérivés alcoylés, en particulier diméthylés, de l'isosorbide, utiles notamment comme solvants dans le cadre de la préparation de compositions pharmaceutiques ou cosmétologiques (brevet US 4,082,881), voire comme principes actifs de compositions d'hygiène buccale (brevet EP 315 334).
- la préparation de dérivés d'isosorbide utilisables dans des compositions détergentes pour carburants (brevet EP 1 106 616),
- la préparation de dérivés alcoylés ou alcénylés utilisables comme plastifiants de polymères, d'adhésifs ou d'encres(brevet WO 99/450060),
- la préparation de biphosphites particuliers utilisables comme agents stabilisants de polymères ou lubrifiants ( brevet FR 2 757 517),
- la préparation d'articles à base d'alcool polyvinylique (brevet US 4,529,666), de polyuréthannes (brevet US 4,383,051), ou de polyesters contenant également des unités monomères de type « téréphthaloyle » (brevets US 3,223,752 et US 6,025,061),
- la préparation de polycondensats comme décrit dans l'article de H. R. KRICHELDORF publié dans le Journal of Macromolecular Science - REV. MACROMOL. CHEM. PHYS., C 37 (4), 599-631 (1997),
- la préparation de polycondensats biodégradables (brevet WO 99/45 054),
- la préparation de laques aqueuses (brevet US 4,418,174) ou de compositions à action recouvrante et/ou colorante de surfaces (brevet US 5,766,679).

Pour la majorité des applications précitées de l'isosorbide et autres produits de déshydratation interne de sucres hydrogénés, en particulier des autres isohexides, il est généralement requis d'appliquer un traitement de purification aux compositions résultant directement de l'étape de déshydratation proprement dite. Ceci, du fait notamment que tout sucre hydrogéné soumis à une telle étape (par exemple le sorbitol) est susceptible, lors de ladite étape, d'être transformé, outre en le produit de déshydratation recherché (par exemple l'isosorbide) en divers co-produits tels que :
- des isomères dudit produit recherché, par exemple des isomères de l'isosorbide tels que l'isomannide et l'isoidide,
- des produits moins déshydratés que le produit recherché ou que ses isomères, par exemple du sorbitan, du mannitan ou de l'iditan,
- des dérivés résultant de l'oxydation ou plus généralement de la dégradation des produits susmentionnés, ces dérivés pouvant inclure, par exemple lorsque le produit recherché est l'isosorbide, des co-produits de type déoxy monoanhydro hexitols, monoanhydro pentitols, monoanhydro tétritols, anhydro hexoses, hydroxyméthyl furfural, ou glycérine,
- des dérivés résultant de la polymérisation des produits susmentionnés, et/ou
- des espèces fortement colorées, de nature mal définie.

Il convient de rappeler que d'une manière générale, tout ou partie de ces différentes catégories de co-produits ou impuretés sont générées peu ou prou lors de l'étape de déshydratation proprement dite du sucre hydrogéné et ce, indépendamment des conditions et précautions mises en oeuvre en pratique lors de ladite étape, et par exemple indépendamment :
- de la nature et de la forme de présentation du catalyseur acide de déshydratation utilisé (acide minéral, acide organique, résine cationique, ...), ou
- de la quantité d'eau ou de solvant(s) organique(s) dans le milieu réactionnel initial, ou
- de la pureté de la composition de sucre hydrogéné, par exemple de sorbitol, utilisée comme matière première.

Différentes technologies ont été préconisées aux fins d'obtenir des compositions issues de ladite étape de déshydratation, par exemple des compositions d'isohexide(s), qui soient améliorées en termes de pureté et ce, de marnière « directe » en jouant sur les conditions réactionnelles lors de ladite étape et/ou de manière « indirecte » en appliquant un ou plusieurs traitement(s) de purification après ladite étape.

Le brevet CA 1 178 288 rappelle en sa page 14, lignes 3-8 qu'il est recommandé de conduire la réaction de déshydratation proprement dite sous une atmosphère de gaz inerte pour éviter des réactions d'oxydation, notamment lorsque des températures et durées de réaction relativement importantes sont envisagées.

Le brevet US 4,861,513 décrit une réaction de déshydratation du sorbitol menée en présence simultanée de gaz inerte (azote) et d'un agent réducteur (hypophosphite de sodium) en vue de la préparation de mélanges de polyols particuliers, lesquels présentent une faible teneur(10 à 26 %) en dianhydrosorbitol.

Pour sa part, le brevet GB 613,444 décrit l'obtention, par déshydratation en milieu eau / xylène, d'une composition d'isosorbide qui est ensuite soumise à un traitement de distillation puis de recristallisation dans un mélange alcool / éther.

Un traitement de purification associant distillation et recristallisation dans un alcool aliphatique inférieur (éthanol, méthanol) est également recommandé récemment dans le brevet WO 00/14081. Ce document indique par ailleurs que dans le cas où la distillation est la seule étape de purification envisagée, il est avantageux de conduire ladite étape en présence de borohydrure de sodium.

Le passage situé page 11, lignes 13-21 dudit brevet décrit la distillation « en atmosphère inerte (argon)» d'une composition d'isosorbide préalablement mise en présence de borohydrure de sodium (NaBH4). Selon le tableau 1 de ce brevet, il apparaît que cette distillation permet d'augmenter la pureté du produit initial (« C-ISOS ») d'une valeur de 98,79 à 99,07 %.

D'autres auteurs ont également préconisé que l'étape de distillation soit menée en présence d'un composé boré, en particulier d'acide borique ou d'une résine anionique préalablement chargée en ions borate, comme décrit dans le brevet US 3,160,641.

Les brevets US 4,408,061 et EP 323,994 envisagent la mise en oeuvre de catalyseurs de déshydratation particuliers (respectivement un halogénure d'hydrogène gazeux et du fluorure d'hydrogène liquide), associés avantageusement à des acides carboxyliques comme co-catalyseurs puis la distillation des compositions brutes d'isosorbide ou d'isomannide ainsi obtenues.

Le brevet US 4,564, 692 mentionne sans aucunement la détailler, la prépurification sur « échangeurs d'ions et/ou charbon actif » de compositions d'isosorbide ou d'isomannide puis, après concentration par évaporation et ensemencement de cristaux de l'isohexide recherché, la cristallisation dans l'eau de celui-ci.

Le brevet EP 380,402 revendique pour sa part la déshydratation de sucres hydrogénés en présence d'hydrogène sous pression et de catalyseur particuliers à base d'une association entre le cuivre et un métal noble du Groupe VIII ou de l'or. Ces conditions sont présentées comme permettant de diminuer significativement la formation d'impuretés de nature polymérique lors de l'étape de déshydratation proprement dite.

Plus récemment, il a été décrit dans le brevet EP 915,091, la possibilité de diminuer encore avantageusement la genèse de tels polymères indésirables et ce, par mise en oeuvre de catalyseurs d'hydrogénation stables aux acides lors de l'étape de déshydratation.

Cependant, les documents précités ne s'intéressent pas spécifiquement aux questions de la stabilité dans le temps des compositions purifiées ainsi obtenues et devant être commercialisées, en particulier de leur stabilité dans le temps dans des conditions classiques de température de stockage, i.e. généralement entre 0 et 40°C.

Ceci résulte du fait que l'isosorbide est généralement considéré, comme souligné en page 600 point 4. de l'article de H. R. KRICHELDORF susmentionné, comme un diol aliphatique particulièrement stable chimiquement et thermiquement.

A l'issue cependant d'une étude approfondie de ces questions, la Société Demanderesse a d'abord fait le double constat selon lequel :
1) non seulement le niveau de stabilité d'une composition telle qu'envisagée ici, par exemple une composition d'isosorbide, n'était pas corrélé à son niveau de pureté,
2) mais encore, la mise en oeuvre d'un agent comme l'azote gazeux ou le borohydrure de sodium telle que décrite dans l'art antérieur, i.e. au plus tard au moment de l'étape de distillation, ne permettait pas d'améliorer significativement cette stabilité.

Et c'est en poursuivant encore ses travaux que la Société Demanderesse a trouvé que de façon surprenante et inattendue, seule la mise en oeuvre a) d'agents stabilisants particuliers, en l'occurrence sous forme non gazeuse et b) à un moment particulier du procédé de préparation, en l'occurrence postérieurement à l'étape proprement dite de distillation, permettait d'atteindre ce but et en particulier de préparer des compositions d'isosorbide dont le comportement au stockage, pour le moins à température ambiante ou modérée, était amélioré. Pour ces travaux, la Société Demanderesse a mis au point un test de stabilité au stockage à 40°C qui sera décrit ultérieurement et qui permet d'évaluer plus rapidement la stabilité de compositions qui, dans la réalité industrielle, sont généralement stockées à température plus faible, i.e. à la température ambiante.

En suite de quoi, la présente invention a pour objet un procédé de préparation d'une composition de produit de déshydratation interne d'un sucre hydrogéné, ledit procédé étant caractérisé par le fait qu'il comprend :
a) une étape de distillation d'un milieu contenant ledit produit de déshydratation interne en vue d'obtenir un distillat enrichi en ce produit,
b) éventuellement, au moins une étape subséquente de purification du distillat ainsi obtenu,
c) une étape subséquente de mise en présence du distillat obtenu lors de l'étape a), puis éventuellement soumis à l'étape b), avec un agent apte à améliorer la stabilité du produit de déshydratation interne majoritairement contenu dans le distillat, le dit agent n'étant pas sous forme gazeuse, et étant choisi dans le groupe comprenant les agents réducteurs, les agents anti-oxydants, les agents capteurs d'oxygène, les agents de stabilisation à la lumière, les agents anti-acides, les agents désactivateurs des métaux et les mélanges quelconques d'au moins deux quelconques de ces produits,
d) éventuellement, une étape subséquente de mise en forme de la composition résultante de produit de déshydratation interne d'un sucre hydrogéné.

L'agent mis en oeuvre lors de l'étape c) subséquente à l'étape a) de distillation proprement dite (ci-après « l'agent d'amélioration») peut être de nature variée.

Conformément à la présente invention, il ne peut consister en un agent de nature gazeuse comme l'azote gazeux, en tous cas uniquement en un tel agent.

La Société Demanderesse a en effet constaté, comme il sera exemplifié par ailleurs, que le fait de stocker une composition d'isosorbide sous une atmosphère inerte tel que de l'azote gazeux sans y introduire un agent d'amélioration conforme à l'invention, ne permettait pas, en fin de compte, d'obtenir une composition d'isosorbide dont la stabilité dans le temps était véritablement améliorée en regard du même produit stocké sous air.

L'ensemble des agents utilisés à l'étape c) peuvent être d'origine naturelle ou synthétique, de nature minérale ou organique.

Par « agents réducteurs » on entend tous les composés ayant des propriétés de donneur d'électron(s).

Ces composés peuvent correspondre à ceux décrits dans le chapitre intitulé « Reduction » en pages 687 à 716 Volume A22 de la 5ème édition de l'ouvrage « Ullmann's Encyclopedia of Industriel Chemistry » (1993) VCH Verlagsgesellschaft mbH, Weinheim (Allemagne).

Il peut s'agir notamment de composés à base de bore ou d'aluminium tels que les hydrures de bore ou d'aluminium décrits en pages 699-703 du chapitre précité et en particulier le borohydrure de sodium (NaBH4) ou l'aluminohydrure de lithium (LiAlH4).

La Société Demanderesse a découvert, 1 comme il sera exemplifié par ailleurs, que contrairement à sa mise en oeuvre avant l'étape a) de distillation, la mise en oeuvre de NaBH₄ au niveau de l'étape c) précitée, i.e après distillation, permettait d'obtenir une composition d'isosorbide dont la stabilité était significativement améliorée.

L'agent réducteur utilisé conformément à l'invention peut également consister en un composé à base de phosphore comme une phosphine ou un phosphite.

L'agent d'amélioration peut également être choisi, comme indiqué ci-avant, parmi les agents anti-oxydants. Par « agents anti-oxydants » on entend en particulier tous les composés aptes, de manière directe ou indirecte, à limiter voire empêcher, quels que soient leurs modes d'action, les phénomènes complexes d'oxydation, y compris d'autooxydation, de substances organiques d'origine naturelle ou synthétique, de nature monomérique ou polymérique.

Ces composés peuvent correspondre à ceux décrits dans le chapitre 1 intitulé « Antioxydants » de la 5ème édition de l'ouvrage « Plastics Additives Handbook » (2001)Carl Hanser Verlag, Münich (Allemagne) ou à tout mélange quelconque d'au moins deux quelconques de ces composés.

De tels composés peuvent agir selon l'un au moins des mécanismes décrits en page 10 à 19 dudit chapitre et peuvent présenter une structure chimique conforme à l'une de celles présentées dans le passage correspondant aux pages 97 à 139 dudit chapitre.

Il peut s'agir en particulier de composés à base d'azote, en particulier des amines, aromatiques ou non, contenant ou non par ailleurs au moins une fonction alcool.

Il peut s'agir notamment d'hydroxylamine, de morpholine, de leurs dérivés et/ou de l'un des composés décrits sous les codes « AO-7 », « AO-15 », « AO-16 », « AO-26 » à « AO-28 » et « AO-35 » à « AO-41 » du passage précité.

L'agent anti-oxydant utilisable comme agent d'amélioration au sens de l'invention peut également consister en un composé aromatique, azoté ou non, contenant ou non au moins une fonction alcool, et consister par exemple, en des composés phénoliques tels que l'hydroquinone, le phénol, les tocophérols et leurs dérivés respectifs et/ou en l'un des composés décrits dans le passage précité sous les codes « AO-1 » à « AO-42 ».

Il peut s'agir en particulier de 2,6-Di-tert.Butyl-4-methylphenol (BHT), d'hydroquinone, ou de mélanges de ceux-ci.

Par « tocophérols et dérivés » on entend notamment l'ensemble des produits décrits dans le brevet WO 99/33776 au nom de la Demanderesse, notamment au niveau du passage situé page 11, ligne 14 à page 12, ligne 11 dudit brevet. Il peut s'agir avantageusement d'un composé constitué en tout ou partie d'α-tocophérol et notamment de vitamine E.

Par ailleurs l'agent d'amélioration utilisé conformément à l'invention peut être constitué d'un composé anti-oxydant à base de phosphore ou de soufre tels que ceux décrits dans le chapitre précité de « Plastics Additives Handbook », par exemple sous les codes « PS-1 » à « PS-12 » et « TS-1 » à « TS-4 ».

Il peut notamment être choisi dans le groupe comprenant les phosphites, les phosphonites, les sulfites, les sels d'esters d'acide thiodipropionique et leurs mélanges.

L'agent d'amélioration au sens de la présente invention peut également consister, comme indiqué précédemment, en un agent de stabilisation à la lumière (« Light Stabilizer »). Cette définition inclut les composés décrits en page 141 à 425 du « Plastics Additives Handbook » précité, en particulier les composés à basé ou non d'azote ou de soufre, aromatiques ou non, contenant ou non au moins une fonction alcool, qui y sont mentionnés, en particulier sous les codes « UVA-1 » à « UVA-27 » et HA(L)S-1 » à « HA(L)S-52 ».

L'agent d'amélioration peut également être choisi parmi les agents anti-acides, cette définition incluant les composés décrits dans le chapitre intitulé « Acide Scavengers » en pages 485 à 510 de l'ouvrage « Plastics Additives Handbook » précité.

Cette définition inclut également les agents alcalins, en particulier ceux qui sont aptes à être utilisés comme « tampons » en milieux neutres ou alcalins et notamment dans des milieux présentant un pH de l'ordre de 7 à 9 environ.

Les agents alcalins peuvent notamment consister en composés, avantageusement en hydroxydes, carbonates, borates ou phosphates, de métaux alcalins et en particulier de sodium ou potassium.

Ces composés de sodium ou potassium peuvent, à titre d'exemple, être choisis dans le groupe comprenant l'hydroxyde de sodium, l'hydroxyde de potassium, le carbonate de sodium, le carbonate de potassium, le métaborate de sodium, le phosphate disodique et les mélanges quelconques de ces produits.

Enfin, ledit agent améliorant peut avantageusement être choisi parmi les agents désactivateurs des métaux, cette définition incluant les composés qualifiés de « Metal Deactivators » tels que décrits en page 18 et 113 dudit ouvrage mais également les composés décrits dans le chapitre intitulé « CHELATING AGENTS » en pages 764 à 795 Volume 5 de la 4ème édition de l'ouvrage « KIRK-OTHMER ENCYCLOPEDIA OF CHEMICAL TECHNOLOGY » (1993)John Wiley & Sons, Inc.

Ces agents désactivateurs des métaux peuvent consister notamment en agents complexants ou chélatants des métaux d'origine naturelle, en particulier contenant au moins une fonction alcool, et notamment obtenus à partir de matières amylacées telles que les gluconates, glucoheptonates, phytates, lactates ou citrates.

La Société Demanderesse a notamment trouvé que ledit agent pouvait avantageusement être apte à complexer ou chélater des métaux tels que le fer, le cuivre, le cobalt, le manganèse ou le nickel. Dans la pratique industrielle, en particulier lors des opérations de fabrication, de mise en forme et de stockage, il est difficile d'éviter toute contamination de métaux dans de telles compositions.

Selon une autre variante de l'invention, en particulier lorsque la composition du produit de déshydratation interne doit être soumise à des contraintes réglementaires sévères, l'agent d'amélioration peut être avantageusement choisi parmi les produits autorisés comme additifs alimentaires, en particulier ceux qualifiés de « anti-oxygènes », « correcteurs d'acidité » ou « séquestrants » au sens de la réglementation européenne, en particulier de la Directive 95/2/CE du Parlement Européen et du Conseil du 20 février 1995 et de ses modifications ultérieures.

L'agent d'amélioration peut alors notamment être choisi dans le groupe comprenant l'acide ascorbique (vitamine C), l'acide érythorbique, l'acide lactique, l'acide citrique, l'acide gallique, les tocophérols, les dérivés (en particulier les sels) de l'ensemble de ces produits, le BHT, le butylhydroxyanisole (BHA) et les mélanges quelconques de ces produits.

L'agent d'amélioration peut être mis en oeuvre au niveau de l'étape c) en de très faibles proportions, à savoir de l'ordre de 0,0001 % (1 ppm) à 2 %, ces pourcentages étant exprimés en poids sec d'agent d'amélioration par rapport au poids sec du produit de déshydratation interne de sucre hydrogéné alors majoritairement présent dans le distillat, par exemple de l'isosorbide alors présent dans ce milieu.

Selon une première variante du procédé selon l'invention, l'agent d'amélioration est utilisé au niveau de l'étape c) dudit procédé à raison de 0,001 à 2 %, de préférence de 0,002 à 1,5 % et encore plus préférentiellement de 0,003 à 1,5 %.

La Société Demanderesse a notamment trouvé que ce taux d'introduction pouvait avantageusement se situer de 0,005 à 1 % par exemple lorsque l'agent d'amélioration consistait en NaBH₄ ou un agent anti-oxydant tel que BHT, morpholine, hydroxylamine, vitamine, C, hydroquinone, erythorbate de sodium ou des composés décrits sous les codes « AO-18 » ou « PS-2 » dans la littérature précitée.

Selon une autre variante du procédé selon l'invention, l'agent d'amélioration est utilisé au niveau de l'étape c) dudit procédé à raison de 0,0001 (1 ppm) à 0,5%, de préférence de 0,0003 (3 ppm) à 0,3 %.

La Société Demanderesse a notamment trouvé que ce taux d'introduction pouvait avantageusement se situer entre 0,0003 (3 ppm) et 0,1 % lorsque l'agent d'amélioration consistait en un agent réducteur comme NaBH₄ ou en un agent anti-acide tel qu'un agent alcalin comme le métaborate de sodium ou le phosphate disodique.

Des taux aussi faibles en agent d'amélioration, par ex de 0,0003 % (3 ppm) à 0,01 % (100 ppm) de NaBH₄ ou d'un autre sel de sodium, peuvent notamment être avantageusement mis en oeuvre aux fins de satisfaire en outre à des contraintes techniques et/ou réglementaires éventuellement existantes en fonction de l'application particulière à laquelle est destinée la composition stabilisée conforme à l'invention.

Dans le cadre du procédé selon l'invention, il convient de préciser que le milieu soumis à l'étape a) de distillation, peut être de nature très variée, y compris en termes de matière sèche, de température et/ou de pureté en le produit de déshydratation recherché.

Il peut s'agir, selon une première variante, d'une composition d'isosorbide consistant en le milieu issu directement de la réaction de déshydratation proprement dite et présentant une pureté en produit recherché, par exemple en isosorbide, de l'ordre de 50 à 80 %.

Selon une autre possibilité, ladite composition peut, du fait notamment qu'elle résulte déjà d'une ou de plusieurs opérations antérieures de purification, notamment par distillation et/ou cristallisation, présenter une pureté en produit recherché, par exemple en isosorbide, supérieure à 80 %.

De manière avantageuse l'étape a) de distillation est suivie d'une étape b)de purification du distillat résultant.

Selon une première variante l'étape b) consiste en une étape de purification selon laquelle le distillat, généralement mis en solution, est traité avec au moins un moyen de purification choisi parmi les moyens de décoloration et les moyens d'échange ionique.

Par « moyens de décoloration », on entend notamment le charbon actif sous forme granulaire ou pulvérulente et les résines d'adsorption.

A titre d'exemple, on peut utiliser, seul ou en combinaison, du charbon actif granulaire comme le produit « CECA DC 50 », du charbon actif pulvérulent comme le produit « NORIT SX + » et/ou une résine comme celles dénommées « DUOLITE XAD 761 », « MACRONET MN-600 » ou « MACRONET MN-400 ».

Par « moyens d'échange ionique », on entend notamment les résines anioniques, faibles ou fortes, et les résines cationiques, faibles ou fortes.

A titre d'exemple, on peut utiliser, seule ou en combinaison, une résine anionique forte comme la résine « AMBERLITE IRA 910 » ou une résine cationique forte comme la résine « PUROLITE C 150 S ».

Le moyen d'échange ionique peut avantageusement comprendre au moins une résine anionique et au moins une résine cationique. De préférence, ce moyen est composé d'un lit mixte de résines anionique(s) et cationique(s) ou d'une succession de résines cationique(s) puis anionique(s) ou d'une succession de résine(s) anionique(s) puis cationique(s).

De manière préférentielle, lors de l'étape b) du procédé conforme à l'invention, le distillat obtenu lors de l'étape a) est traité dans un ordre quelconque par au moins un charbon actif et par au moins une résine, ionique ou non ionique.

De manière très avantageuse, ledit distillat est traité d'abord par un charbon actif puis par au moins une résine puis de nouveau par un charbon actif.

Selon une autre variante du procédé selon l'invention, la Société Demanderesse a trouvé qu'il était particulièrement avantageux que la composition soumise à l'étape b) de purification présente déjà certaines caractéristiques en termes de teneur maximale en impuretés particulières, par exemple en acide formique et en espèces de type « monoanhydrohexoses ».

Elle a trouvé par ailleurs qu'une telle teneur pouvait notamment être garantie en soumettant directement le distillat obtenu lors de l'étape a) à ladite étape b) de purification.

Le procédé selon l'invention peut donc être caractérisé par le fait que le distillat soumis à ladite étape b) présente une teneur en acide formique inférieure à 0,002 % (20 ppm) et une teneur en monoanhydrohexoses inférieure à 0,02 % (200 ppm), ces pourcentages étant exprimés en poids sec par rapport au poids sec du produit de déshydratation interne de sucre hydrogéné alors majoritairement présent dans ledit distillat, par exemple au poids sec d'isosorbide présent dans ledit distillat.

Le distillat peut notamment présenter une teneur en acide formique inférieure à 0,0005 % (5 ppm) et une teneur en monoanhydrohexoses inférieure à 0,005 % (50 ppm).

Selon une autre variante et de manière avantageuse, l'agent d'amélioration utilisé selon l'invention est mis en oeuvre directement après l'étape b) de purification et notamment, comme il sera exemplifié par ailleurs, introduit directement sur la solution aqueuse purifiée de produit de déshydratation interne issue de l'étape b), laquelle présente généralement une température au plus égale à 60°C.

Quel que soit le mode de fonctionnement du procédé objet de l'invention, la Société Demanderesse a également trouvé qu'il pouvait être avantageux que tout ou partie de l'étape c), au cours de laquelle il y a présence de l'agent d'amélioration, se fasse au sein d'un milieu qui soit liquide et dont la température soit au moins égale à la température de ramollissement ou de fusion du produit de déshydratation interne recherché (par exemple l'isosorbide) mais inférieure à 140°C environ.

Cette température peut, en particulier dans le cas de l'isosorbide, se situer entre 60 et 135°C.

Ces conditions améliorent la répartition homogène de l'agent d'amélioration au sein de la composition résultante, en particulier si celle-ci doit être refroidie puis mise en forme conformément à l'étape d) facultative.

A ce titre, la Société Demanderesse pense qu'il peut être avantageux que ledit agent d'amélioration présente une solubilité minimale dans l'eau et/ou dans le produit de déshydratation interne recherché.

En particulier, la présente invention peut être également caractérisée par le fait que ledit agent d'amélioration présente une solubilité, dans l'eau à 20°C, au moins égale à 0,01 %, de préférence au moins égale à 0,1 %.

Après l'étape c) et comme indiqué précédemment, la composition de produit de déshydratation interne d'un sucre hydrogéné obtenue selon l'invention peut être mise en forme lors d'une étape d) ultérieure.

Cette étape peut consister en une opération de pastillage ou d'écaillage de la masse cristallisée ou « massé » résultant du refroidissement, notamment par contact sur une surface froide, de la composition issue de l'étape c).

L'étape d) de mise en forme peut, si on le souhaite, être suivie d'une étape de broyage et/ou de tamisage et ce, avant toute étape de stockage et/ou d'ensachage de la composition ainsi obtenue.

Selon une autre variante, la composition de produit de déshydratation interne d'un sucre hydrogéné obtenue selon l'invention peut, après l'étape c), être stockée en l'état, en particulier à l'état liquide ou pâteux, sans étape spécifique de mise en forme ultérieure.

La composition résultant du procédé selon l'invention peut par ailleurs avoir subi, à tout moment, une étape de concentration, en particulier une étape d'évaporation sous vide, ladite étape étant conduite dans les conditions les plus douces possible, notamment en termes de durée et de température.

Selon une première variante, ladite étape de concentration est menée en tout ou partie simultanément à l'étape c) conforme à l'invention.

Selon une autre variante, ladite étape de concentration est menée, en tout ou partie, antérieurement à l'étape c) conforme à l'invention, de préférence immédiatement avant ladite étape c).

Dans tous les cas, il est avantageux de mener ladite étape de concentration directement après l'étape b) de purification, dès lors que cette étape b) a été menée.

En suite de quoi, quel que soit le moment où une étape de concentration au moins a été opérée dans le cadre du procédé conforme à l'invention, y compris donc antérieurement à l'étape c), ledit procédé permet de préparer une composition liquide stable de produit de déshydratation interne d'un sucre hydrogéné, en particulier une composition liquide stable d'isosorbide, dont la matière sèche (« MS ») est élevée, i.e. comprise entre 50 et 90 %, notamment comprise entre 75 et 88 %.

Cette MS peut notamment être de l'ordre de 85 % environ.

L'ensemble des étapes du procédé selon l'invention, obligatoires ou facultatives, qui viennent d'être décrites peuvent en outre, si on le souhaite, être menées sous atmosphère inerte, y compris l'étape c) caractéristique de la présente invention et/ou toute étape ultérieure, notamment de mise en forme, de stockage ou d'ensachage.

En suite de quoi, on dispose d'un nouveau moyen apte à fournir une composition d'un produit de déshydratation interne d'un sucre hydrogéné, par exemple une composition d'isosorbide, dont la stabilité est améliorée, une telle stabilité étant requise quelles que soient les utilisations auxquelles on destine ladite composition et quelle que soit par ailleurs la pureté de ladite composition.

Il est remarquable de souligner que le procédé selon l'invention permet d'obtenir en particulier une composition du genre en question qui bien que présentant une pureté certes élevée mais non optimale, se révèle être stable. Par « composition stable » au sens de la présente invention, on entend une composition qui, stockée dans une atmosphère non inerte pendant une durée d'au moins un mois et à une température de 40°C, présente à la fois une teneur en acide formique inférieure à 0,0005 % (5 ppm) et une teneur globale en monoanhydrohexoses inférieure à 0,005 % (50 ppm), ces pourcentages étant exprimés en poids sec par rapport au poids sec de ladite composition. Cette stabilité peut bien évidemment être très significativement supérieure à 1 mois et atteindre au moins deux mois, de préférence au moins 6 mois et plus préférentiellement encore au moins un an ; ce qui signifie que dans des conditions de stockage habituelles, i.e. à température ambiante, les compositions selon l'invention pourront être stockées pendant des durées significativement plus élevées que celles susmentionnées.

On dispose désormais, entre autres, d'un produit consistant en une composition d'isosorbide ou autre produit de déshydratation interne d'un sucre hydrogéné, qui est stable selon la définition qui vient d'être donnée et qui, simultanément, présente une pureté inférieure à 99,8 %, se situant notamment entre 98,0 et 99,7 % environ.

De manière remarquable, ladite composition présente une stabilité d'au moins deux mois, de préférence d'au moins six mois, et encore plus préférentiellement d'au moins un an.

La Société demanderesse a notamment trouvé qu'une composition conforme à l'invention était tout particulièrement apte à présenter une telle stabilité dès lors qu'elle présentait un pH supérieur à 5, de préférence supérieur à 6 et notamment compris entre 6,5 et 9,5 environ.

Ceci, quelles que soient sa forme de présentation et sa matière sèche.

De manière particulièrement avantageuse, ladite composition présente un pH compris entre 7 et 9 environ ; la mesure du pH se faisant après ajustement de la matière sèche de ladite composition à une valeur de 40 % et ce, par tout moyen de dilution ou de concentration à la portée de l'homme de l'art.

Les nouvelles compositions selon l'invention sont caractérisées par le fait qu'elles contiennent de 0,0003 à 0,01% d'un agent d'amélioration tel que décrit précédemment, ces pourcentages étant exprimés en poids sec d'agent d'amélioration par rapport au poids sec du produit de déshydratation interne de sucre hydrogéné, par exemple l'isosorbide, majoritairement présent dans la composition.

De telles compositions peuvent notamment être utilisées pour la préparation de produits ou mélanges, polymériques ou non, biodégradables ou non, destinés aux industries chimiques, pharmaceutiques, cosmétologiques ou alimentaires.

La présente invention va être décrite de façon encore plus détaillée à l'aide des exemples qui suivent et qui ne sont aucunement limitatifs.

### EXEMPLE 1

Dans un réacteur agité double enveloppe, on introduit 1 kg d'une solution de sorbitol à 70 % de MS commercialisée par la Demanderesse sous l'appellation « NEOSORB^{®} 70/02 » et 7 g d'acide sulfurique concentré. Le mélange obtenu est chauffé sous vide (pression d'environ 100 mbars) pendant 5 heures de façon à éliminer l'e contenue dans le milieu réactionnnel initial et celle provenant de la réaction de déshydratation du sorbitol.

Le brut réactionnel est ensuite refroidi vers 100°C puis neutralisé avec 11,4 g d'une solution de soude à 50 %. La composition d'isosorbide ainsi neutralisée est ensuite distillée sous vide (pression inférieure à 50 mbars).

Le distillat brut d'isosorbide, légèrement coloré (couleur jaune claire) est ensuite mis en solution dans de l'eau distillée de façon à obtenir une solution à 40 % de MS.

Cette solution est ensuite percolée sur une colonne de charbon actif granulaire « CECA DC 50 » à une vitesse de 0,5 BV/h (« Bed Volume » ou « Volume de lit »/heure). La composition d'isosorbide décolorée ainsi obtenue est ensuite passée, à une vitesse de 2 BV/h successivement sur une colonne de résine cationique forte « PUROLITE C 150 S » puis une colonne de résine anionique forte « AMBERLITE IRA 910 ».

La solution d'isosorbide est ensuite directement concentrée sous vide. La masse fondue obtenue cristallise au refroidissement sous forme d'un « massé » de gros cristaux que l'on broye ensuite pour obtenir une poudre de couleur blanche présentant une humidité de 0,3 %.

Ses teneurs en, respectivement, acide formique et monoanhydrohexoses sont inférieures à 0,0005 % (5 ppm), exprimées chacune en poids sec par rapport au poids sec de ladite composition.

20 g de ce massé d'isosorbide sont directement introduits dans un récipient en verre d'une contenance de 50 ml qui, après avoir été fermé hermétiquement, est placé dans une étuve maintenue à 40°C.

Après 1 mois de stockage dans ces conditions, la composition d'isosorbide présente une teneur en acide formique de 1000 ppm et une teneur en monoanhydrohexones de 7000 ppm environ.

Le « massé » d'isosorbide ainsi testé ne peut donc pas être considéré comme stable, au sens de la présente invention.

### EXEMPLE 2

Dans cet exemple, conforme à l'invention, on récupère une fraction de la solution d'isosorbide directement issue, après distillation, du traitement de passage sur charbon actif puis résines tel que décrit au niveau de EXEMPLE 1, solution dont la matière sèche et la pureté en isosorbide ont été mesurées.

Dès sa récupération, on introduit dans ladite fraction une quantité de NaBH₄ correspondant à 0,005 % (50 ppm) en poids du poids d'isosorbide contenu dans ladite fraction (sec/sec) puis on opère directement l'étape de concentration et ce, dans les mêmes conditions que celles décrites dans l'EXEMPLE 1.

Le massé obtenu directement après refroidissement présente des caractéristiques de pureté (99,1 % environ) et de teneur en acide formique et monoanhydrohexoses qui sont logiquement du même ordre que celles du massé obtenu lors de l'EXEMPLE 1.

Cependant, après 1 mois de stockage dans les mêmes conditions que celles décrites à l'EXEMPLE 1, la composition d'isosorbide ainsi adjuvantée de NaBH4 après distillation, présente toujours des teneurs respectives en acide formique et monoanhydrohexoses inférieures à 5 ppm.

Cette composition peut donc être considérée comme stable au sens de la présente invention.

Des mesures complémentaires ont d'ailleurs montré qu'après plus de 6 mois de stockage dans les mêmes conditions, cette même composition présentait encore une teneur en acide formique inférieure à 5 ppm et une teneur en monoanhydrohexoses significativement inférieure à 50 ppm. En suite de quoi cette composition présente une stabilité d'au moins six mois au sens de l'invention.

### EXEMPLE 3

Dans cet exemple, non conforme à l'invention, on récupère une fraction du brut réactionnel directement issu de l'étape de neutralisation tel que décrit dans l'EXEMPLE 1, brut réactionnel neutralisé dont la matière sèche et la pureté en isosorbide ont été mesurées.

Dès sa récupération, on introduit dans ladite fraction une quantité de NaBH₄ correspondant à 0,02 % (200 ppm) de l'isosorbide contenu dans ladite fraction (sec / sec) puis on opère directement les étapes de distillation sous vide, de purification sur charbon actif puis résines, de concentration et de refroidissement telles que décrites au niveau de l'EXEMPLE 1.

Le massé obtenu directement après refroidissement présente des caractéristiques de pureté et de teneur en acide formique et monoanhydrohexoses qui sont du même ordre que celles du massé obtenu lors de l'EXEMPLE 2.

Cependant, après 1 mois de stockage dans les mêmes conditions que celles décrites dans l'EXEMPLE 1, la composition d'isosorbide ainsi obtenue présente une teneur en acide formique de 1200 ppm et une teneur en monoanhydrohexoses de 5000 ppm environ.

Cet exemple montre que la mise en oeuvre d'un produit comme NaBH₄ avant l'étape de distillation proprement dite ne permet pas, en fin de compte, de conférer à la composition d'isosorbide résultante un caractère de stabilité, au sens de la présente invention.

### EXEMPLE 4

Dans cet exemple, non conforme à l'invention, on obtient un massé dans les mêmes conditions que celles de l'EXEMPLE 1 si ce n'est que l'ensemble des étapes postérieurs à l'étape b) de purification sont menées en atmosphère inerte, sous azote.

Le massé présente des caractéristiques de pureté et de teneur en acide formique et monoanhydrohexoses qui sont du même ordre que celles du massé obtenu dans l'EXEMPLE 1.

Cependant, après 1 mois de stockage dans les mêmes conditions que celles de l'EXEMPLE 1, la composition d'isosorbide ainsi obtenue présente une teneur en acide formique de 700 ppm et en monoanhydrohexoses de 6000 ppm environ.

Cet exemple montre que la seule mise en oeuvre d'azote gazeux et ce, même après l'étape de distillation, ne permet pas, en fin de compte, de conférer à la composition d'isosorbide résultante un caractère de stabilité, au sens de la présente invention.

### EXEMPLE 5

Dans cet exemple, conforme à l'invention, on opère comme décrit dans l'EXEMPLE 2, si ce n'est que :
1) après son passage sur charbon actif puis résines, le distillat est traité avec 5 % (sec/sec) de charbon actif en poudre « NORIT SX + » à 20°C pendant 1 heure, et,
2) on introduit directement dans le distillat ainsi traité, avant concentration, 50 ppm de respectivement NaBH₄, morpholine, BHT ou vitamine C.

Chacun des quatre massés obtenus directement après les étapes de concentration et refroidissement a été stocké dans les mêmes conditions que celles décrites dans l'EXEMPLE 1.

Après 1 ou 2 mois de stockage à 40°C, aucun de ces quatre produits n'a vu sa teneur en acide formique atteindre la valeur de 5 ppm ni sa teneur en monoanhydrohexoses atteindre la valeur de 50 ppm.

De manière remarquable, des tests menés pendant 6 mois et plus dans les mêmes conditions sur les massés issus des distillats traités par NaBH₄ ou morpholine ont par ailleurs montré que cette stabilité pouvait être maintenue au moins pendant cette durée.

On dispose ainsi de compositions d'isosorbide présentant une stabilité d'au moins deux mois, en particulier d'au moins six mois.

### EXEMPLE 6

On prépare une solution décolorée et purifiée d'isosorbide comme décrit au niveau de l'EXEMPLE 1.

Cette solution est, après passage sur résine cationique forte puis résine anionique forte, directement concentrée sous vide et ce, jusqu'à une matière sèche de 85 % environ.

Le pH de cette composition d'isosorbide, mesuré après ajustement de sa matière sèche à une valeur de 40 % par dilution dans l'eau distillée, est de l'ordre de 5.0.

Il a alors été constaté, lors d'un test de stockage effectué comme décrit précédemment à 40°C, qu'une telle composition ne pouvait pas être considérée comme stable au sens de la présente invention.

On prépare des compositions d'isosorbide conformes à l'invention par introduction, dans une solution d'isosorbide décolorée, purifiée et concentrée à 85 % telle que décrite ci-avant et dès après l'étape de concentration, de quantités faibles d'agents d'amélioration consistant respectivement en :
- 0,0002 % (2 ppm) de NaBH₄, ou
- 0,0010 % (10 ppm) de NaBH₄, ou
- 0,0005 % (5 ppm) de NaOH, ou
- 0,0012 % (12 ppm) de NaBO₂, ou
- 0,0015 % (15 ppm) de Na₂HPO₄.

Le pH des compositions ainsi obtenues varie de 6,6 à 8,6 environ.

La Société Demanderesse a alors observé, lors de tests de stockage effectués à 40°C comme décrit précédemment, que l'ensemble de ces compositions pouvaient non seulement être considérées comme stables au sens de la présente invention mais encore que cette stabilité était d'au moins deux mois.

Il a d'ores et déjà été vérifié au niveau de certaines desdites compositions que cette stabilité était d'au moins trois mois, voire six mois ou un an.

En suite de quoi on constate que la mise en oeuvre de très faibles quantités d'agents d'amélioration, i.e. au plus égales à 0,1 % (sec/sec), notamment comprises entre 0,0001 % (1 ppm) et 0,01 % (100 ppm) et y comprises inférieures à 0,005 % (50 ppm), permettait de disposer d'un moyen simple et peu coûteux de stabilisation de compositions du genre en question, ce moyen étant par ailleurs généralement apte à répondre aux contraintes techniques et/ou réglementaires auxquelles peuvent être soumises lesdites compositions. Sans vouloir être liée par une quelconque théorie, la Société Demanderesse considère que l'effet engendré par une telle mise en oeuvre sur le pH desdites compositions peut, directement ou non, jouer, au moins partiellement, un rôle dans l'augmentation de la stabilité desdites compositions, en tous cas de leur stabilité spécifique à température ambiante ou modérée (40°C).

## Revendications

1. Procédé de préparation d'une composition de produit de déshydratation interne d'un sucre hydrogéné, **caractérisé par le fait qu'**il comprend :
a)une étape de distillation d'un milieu contenant ledit produit de déshydratation interne en vue d'obtenir un distillat enrichi en ce produit,
b)êventuellement, au moins une étape subséquente de purification du distillat ainsi obtenu,
c)une étape subséquente de mise en présence du distillat obtenu lors de l'étape a) puis éventuellement soumis à l'étape b), avec un agent apte à améliorer la stabilité du produit de déshydratation interne majoritairement contenu dans le distillat, ledit agent n'étant pas sous forme gazeuse, et étant choisi dans le groupe comprenant les agents réducteurs, les agents anti-oxydants, les agents capteurs d'oxygène, les agents de stabilisation à la lumière, les agents anti-acides, les agents désactivateurs des métaux et les mélanges quelconques d'au moins deux quelconques de ces produits
d)éventuellement, une étape subséquente de mise en forme de la composition résultante de produit de déshydratation interne d'un sucre hydrogéné.

2. Procédé selon la revendication 1, **caractérisé par le fait que** l'agent mis en oeuvre au niveau de l'étape c) est choisi dans le groupe comprenant les composés à base de bore, les composés à base de sodium, les composés à base de potassium, les composés à base de phosphore, les composés à base d'azote, les composés à base de soufre, les composés aromatiques, les composés contenant au moins une fonction alcool et les mélanges quelconques d'au moins deux quelconques de ces produits

3. Procédé selon l'une quelconque des revendications 1 à 2, **caractérisé par le fait que** l'agent mis en oeuvre au niveau de l'étape c) est utilisé à raison de 0,0001 à 2 %, , ces pourcentages étant exprimés en poids sec dudit agent par rapport au poids sec du produit de déshydratation interne de sucre hydrogéné majoritairement présent dans le distillat.

4. Procédé selon la revendication 3, **caractérisé par le fait que** l'agent est utilisé à raison de 0,001 à 2 %, de préférence de 0,002 à 1,5 % et encore plus préférentiellement de 0,003 à 1,5 %.

5. Procédé selon la revendication 4, **caractérisé par le fait que** l'agent est utilisé à raison de 0,005 à 1 %.

6. Procédé selon la revendication 5, **caractérisé par le fait que** l'agent consiste en borohydrure de sodium (NaBH₄) ou en un agent anti-oxydant.

7. Procédé selon la revendication 3, **caractérisé par le fait que** l'agent est utilisé à raison de 0,0001 à 0,5 % de préférence de 0,0003 à 0,3 %.

8. Procédé selon la revendication 7, **caractérisé par le fait que** l'agent est utilisé à raison de 0,0003 à 0, 1 %.

9. Procédé selon la revendication 8, **caractérisé par le fait que** l'agent est utilisé à raison de 0,0003 à 0,01 %.

10. Procédé selon l'une quelconque des revendications 8 et 9, **caractérisé par le fait que** l'agent consiste en un agent réducteur ou en un agent anti-acide, l'agent anti-acide étant de préférence choisi parmi les agents alcalins.

11. Procédé selon la revendication 10, **caractérisé par le fait que** l'agent est un sel de sodium.

12. Procédé selon l'une quelconque des revendications 1 à 11, **caractérisé par le fait que** l'agent mis en oeuvre au niveau de l'étape c) présente une solubilité, dans l'eau à 20°C, au moins égale à 0,01 %, de préférence au moins égale à 0,1 %.

13. Procédé selon l'une quelconque des revendications 1 à 12, **caractérisé par le fait que** l'étape a) de distillation est suivie d'une étape b) de purification, de préférence une étape b) de purification au cours de laquelle le distillat est traité, dans un ordre quelconque, par au moins un charbon actif et par au moins une résine, ionique ou non ionique.

14. Procédé selon l'une quelconque des revendications 1 à 13, **caractérisé par le fait qu'**il comprend au moins une étape de concentration menée, en tout ou partie, antérieurement ou simultanément à l'étape c).

15. Composition contenant au moins un produit de déshydratation interne d'un sucre hydrogéné, **caractérisée par le fait qu'**elle contient de 0,0003 à 0,01 %, d'un agent apte à améliorer la stabilité du produit de deshydratation interne choisi dans le groupe comprenant les agents réducteurs, les agents anti-oxydants, les agents capteurs d'oxygène, les agents de stabilisation à la lumière, les agents anti-acides, les agents désactivateurs des métaux et des mélanges quelconques d'au moins deux quelconques de ces produits, ces pourcentages étant exprimés en poids sec dudit agent par rapport au poids sec du produit de déshydratation interne de sucre hydrogéné majoritairement présent dans ladite composition.

16. Composition selon la revendication 16, **caractérisée par le fait qu'**elle contient de 0,0003 à 0,01 %, d'un agent réducteur ou d'un agent anti-acide, l'agent anti-acide étant de préférence choisi parmi les agents alcalins.

17. Composition selon l'une quelconque des revendications 15 à 16, **caractérisée en ce qu'**il s'agit d'une composition d'isosorbide.

18. Composition selon l'une quelconque des revendications 15 à 17, **caractérisée par le fait qu'**elle présente un pH supérieur à 5, de préférence supérieur à 6 et plus préférentiellement compris entre 6,5 et 9,5 environ.

19. Composition selon la revendication 18, **caractérisée par le fait qu'**elle présente d'un pH compris entre 7 et 9 environ.

20. Composition selon l'une quelconque des revendications 15 à 19, **caractérisée en ce qu'**elle présente une matière sèche comprise entre 50 et 90 %, de préférence comprise entre 75 et 88 %.

21. Utilisation d'une composition selon l'une quelconque des revendications 15 à 20,
dans la préparation de produits et mélanges, polymériques ou non, biodégradables ou non, destinés en particulier aux industries chimiques, pharmaceutiques, cosmétologiques ou alimentaires.

## Claims

1. Method for preparing a composition of an internal dehydration product of a hydrogenated sugar, **characterized by** the fact that it comprises:
(a) a step of distilling a medium containing the said internal dehydration product in order to obtain a distillate enriched in this product,
(b) optionally, at least one subsequent step of purifying the distillate thus obtained,
(c) a subsequent step of bringing the distillate obtained during step a), and then optionally subjected to step b), into contact with an agent capable of improving the stability of the internal dehydration product predominantly contained in the distillate, the said agent not being in gaseous form, and being selected from the group consisting of reducing agents, antioxidants, oxygen scavengers, light stabilizers, anti-acid agents, metal-deactivating agents and any mixtures of at least any two of these products,
(d) optionally, a subsequent step of shaping the resulting composition of an internal dehydration product of a hydrogenated sugar.

2. The method according to claim 1, **characterized by** the fact that the agent used in step (c) is selected from the group consisting of compounds based on boron, compounds based on sodium, compounds based on potassium, compounds based on phosphorus, compounds based on nitrogen, compounds based on sulphur, aromatic compounds, compounds containing at least one alcohol functional group and any mixtures of at least any two of these products.

3. The method according to claim 1 to 2, **characterized by** the fact that the agent used in step (c) is used in an amount of 0.0001 to 2%, these percentages being expressed by dry weight of the said agent relative to the dry weight of the internal dehydration product of a hydrogenated sugar predominantly present in the distillate.

4. The method according to claim 3, **characterized by** the fact that the agent is used in an amount of 0.001 to 2%, preferably of 0.002 to 1.5 %, and still more preferably of 0.003 to 1.5 %.

5. The method according to claim 4, **characterized by** the fact that the agent is used in an amount of 0.005 to 1%.

6. The method according to claim 5, **characterized by** the fact that the agent consists of sodium borohydride (NaBH₄) or an antioxidant.

7. The method according to claim 3, **characterized by** the fact that the agent is used in an amount of 0.0001 to 0.5%.

8. The method according to claim 7, **characterized by** the fact that the agent is used in an amount of 0.0003 to 0.1 %.

9. The method according to claim 8, **characterized by** the fact that the agent is used in an amount of 0.0003 to 0.01%.

10. The method according to any of claims 8 and 9, **characterized by** the fact that the agent consists of a reducing agent or of an anti-acid agent, the anti-acid agent being preferably selected from alkaline agents.

11. The method according to claim 10, **characterized by** the fact that the agent is a sodium salt.

12. The method according to any of claims 1 to 11, **characterized by** the fact that the agent used in step (c) has a solubility in water at 20°C at least equal to 0.01 %, preferably at least equal to 0.1 %.

13. The method according to any of claims 1 to 12, **characterized by** the fact that step a) of distillation is followed by a step b) of purification, preferably a step b) of purification during which the distillate is treated, in any order, with at least one activated charcoal and with at least one ionic or non ionic resin.

14. The method according to any of claims 1 to 13, **characterized by** the fact that it comprises at least one concentration step carried out, completely or partly, prior to or simultaneously with step c).

15. Composition containing at least one internal dehydration product of a hydrogenated sugar, **characterized by** the fact that it contains from 0.0003 to 0.01 % of an agent capable of improving the stability of the internal dehydration product, selected from the group consisting of reducing agents, anti-oxidants, oxygen scavengers, light stabilizers, anti-acid agents, metal-deactivating agents and any mixtures of at least any two of these products, these percentages being expressed by dry weight of the said agent relative to the dry weight of the internal dehydration product of a hydrogenated sugar predominantly present in the said composition.

16. Composition according to claim 15, **characterized by** the fact that it contains from 0.0003 to 0.01 % of a reducing agent or of an anti-acid agent, said anti-acid agent being preferably selected from alkaline agents.

17. Composition according to any of claims 15 to 16, **characterized by** the fact that it is an isosorbide composition.

18. Composition according to any of claims 15 to 17, **characterized by** the fact that it has a pH value higher than 5, preferably higher than 6 and more preferably of between 6.5 and 9.5.

19. Composition according to claim 18, **characterized by** the fact that it has a pH of between about 7 and 9.

20. Composition according to any of claims 15 to 19, **characterized by** the fact that is has a dry matter content of between 50 and 90 %, preferably of between 75 and 88 %.

21. Use of a composition according to any of claims 15 to 20, for the preparation of polymeric or non polymeric, biodegradable or non biodegradable products and mixtures, usable in particular in chemical, pharmaceutical, cosmetic and food industry.

## Patentansprüche

1. Verfahren zur Herstellung einer Zusammensetzung eines internen Dehydratisierungsproduktes eines hydrierten Zuckers, **dadurch gekennzeichnet, dass** es umfasst:
a) einen Schritt der Destillation eines Mediums, das das interne Dehydratisierungsprodukt enthält, um ein mit diesem Produkt angereichertes Destillat zu erhalten,
b) gegebenenfalls mindestens einen anschließenden Schritt der Reinigung des so erhaltenen Destillats,
c) einen anschließenden Schritt des in Kontakt Bringens des in Schritt a) erhaltenen, dann gegebenenfalls Schritt b) unterzogenen, Destillats mit einem Mittel, das die Stabilität des internen Dehydratisierungsproduktes, das hauptsächlich in dem Destillat vorhanden ist, verbessern kann, wobei das Mittel nicht gasförmig ist und ausgewählt ist aus der Gruppe, umfassend Reduktionsmittel, Antioxidantien, Sauerstoffifänger, Lichtstabilisatoren, Antisäuremittel, Metall-Deaktivierungsmittel und irgendwelchen Mischungen von mindestens irgendwelchen zweien dieser Produkte,
d) gegebenenfalls einen anschließenden Schritt des Formens der resultierenden Zusammensetzung eines internen Dehydratisierungsproduktes eines hydrierten Zuckers.

2. Verfahren gemäß Anspruch 1, **dadurch gekennzeichnet, dass** das in Schritt c) verwendete Mittel aus der Gruppe, umfassend Verbindungen auf der Basis von Bor, Verbindungen auf der Basis von Natrium, Verbindungen auf der Basis von Kalium, Verbindungen auf der Basis von Phosphor, Verbindungen auf der Basis von Stickstoff, Verbindungen auf der Basis von Schwefel, aromatischen Verbindungen, Verbindungen, die mindestens eine Alkoholfunktion enthalten, und irgendwelchen Mischungen von mindestens irgendwelchen zweien dieser Produkte, ausgewählt ist.

3. Verfahren gemäß einem der Ansprüche 1 bis 2, **dadurch gekennzeichnet, dass** das in Schritt c) verwendete Mittel in einer Menge von 0,0001 bis 2% verwendet wird, wobei diese Prozentangaben als Trockengewicht dieses Mittels, bezogen auf das Trockengewicht des internen Dehydratisierungsproduktes eines hydrierten Zuckers, das hauptsächlich in dem Destillat vorhanden ist, ausgedrückt sind.

4. Verfahren gemäß Anspruch 3, **dadurch gekennzeichnet, dass** das Mittel in einer Menge von 0,001 bis 2%, bevorzugt von 0,002 bis 1,5% und noch stärker bevorzugt von 0,003 bis 1,5% verwendet wird.

5. Verfahren gemäß Anspruch 4, **dadurch gekennzeichnet, dass** das Mittel in einer Menge von 0,005 bis 1 % verwendet wird.

6. Verfahren gemäß Anspruch 5, **dadurch gekennzeichnet, dass** das Mittel aus Natriumborhydrid (NaBH₄) oder aus einem Antioxidans besteht.

7. Verfahren gemäß Anspruch 3, **dadurch gekennzeichnet, dass** das Mittel in einer Menge von 0,0001 bis 0,5%, bevorzugt von 0,0003 bis 0,3% verwendet wird.

8. Verfahren gemäß Anspruch 7, **dadurch gekennzeichnet, dass** das Mittel in einer Menge von 0,0003 bis 0, 1 % verwendet wird.

9. Verfahren gemäß Anspruch 8, **dadurch gekennzeichnet, dass** das Mittel in einer Menge von 0,0003 bis 0,01 % verwendet wird.

10. verfahren gemäß einem der Ansprüche 8 und 9, **dadurch gekennzeichnet, dass** das Mittel aus einem Reduktionsmittel oder aus einem Antisäuremittel besteht, wobei das Antisäuremittel bevorzugt aus alkalischen Mitteln ausgewählt ist.

11. Verfahren gemäß Anspruch 10, **dadurch gekennzeichnet, dass** das Mittel ein Natriumsalz ist.

12. Verfahren gemäß einem der Ansprüche 1 bis 11, **dadurch gekennzeichnet, dass** das in Schritt c) verwendete Mittel eine Löslichkeit in Wasser bei 20°C von mindestens gleich 0,01%, bevorzugt von mindestens gleich 0, 1 % aufweist.

13. Verfahren gemäß einem der Ansprüche 1 bis 12, **dadurch gekennzeichnet, dass** auf den Schritt a) der Destillation ein Schritt b) der Reinigung folgt, bevorzugt ein Schritt b) der Reinigung, während dem das Destillat in irgendeiner Reihenfolge mit mindestens einer Aktivkohle und mit mindestens einem ionischen oder nichtionischen Harz behandelt wird.

14. Verfahren gemäß einem der Ansprüche 1 bis 13, **dadurch gekennzeichnet, dass** es mindestens einen Konzentrierungsschritt umfasst, der vollständig oder teilweise, vor oder gleichzeitig mit Schritt c) durchgeführt wird.

15. Zusammensetzung, enthaltend mindestens ein internes Dehydratisierungsprodukt eines hydrierten Zuckers, **dadurch gekennzeichnet, dass** sie von 0,0003 bis 0,01 % eines Mittels enthält, das die Stabilität des internen Dehydratisierungsproduktes, verbessern kann, ausgewählt aus der Gruppe, umfassend Reduktionsmittel, Antioxidantien, Sauerstofffänger, Lichtstabilisatoren, Antisäuremittel, Metall-Deaktivierungsmittel und irgendwelchen Mischungen von mindestens irgendwelchen zweien dieser Produkte, wobei diese Prozentangaben als Trockengewicht dieses Mittels, bezogen auf das Trockengewicht des internen Dehydratisierungsproduktes eines hydrierten Zuckers, das hauptsächlich in der Zusammensetzung vorhanden ist, ausgedrückt sind.

16. Zusammensetzung gemäß Anspruch 15, **dadurch gekennzeichnet, dass** sie von 0,0003 bis 0,01 % eines Reduktionsmittels oder eines Antisäuremittels enthält, wobei das Antisäuremittel bevorzugt aus alkalischen Mitteln ausgewählt ist.

17. Zusammensetzung gemäß einem der Ansprüche 15 bis 16, **dadurch gekennzeichnet, dass** es sich um eine Isosorbid-Zusammensetzung handelt.

18. Zusammensetzung gemäß einem der Ansprüche 15 bis 17, **dadurch gekennzeichnet, dass** sie einen pH-Wert von größer als 5 aufweist, bevorzugt von größer als 6 und noch stärker bevorzugt von etwa zwischen 6,5 und 9,5.

19. Zusammensetzung gemäß Anspruche 18, **dadurch gekennzeichnet, dass** sie einen pH-Wert von etwa zwischen 7 und 9 aufweist.

20. Zusammensetzung gemäß einem der Ansprüche 15 bis 19, **dadurch gekennzeichnet, dass** sie zwischen 50 und 90%, bevorzugt zwischen 75 und 88% Trockensubstanz aufweist.

21. Verwendung einer Zusammensetzung gemäß einem der Ansprüche 15 bis 20 zur Herstellung von polymeren oder nicht-polymeren, biologisch abbaubaren oder nicht biologisch abbaubaren Produkten und Mischungen, die insbesondere für die chemischen, pharmazeutischen, Kosmetik- oder Nahrungsmittelindustrien bestimmt sind.
